# NEW EUROPEAN PATENT SPECIFICATION

(11) **EP 0 649 537 B2**
(45) Date of publication and mention of the opposition decision: **22.02.2006**
(45) Mention of the grant of the patent: 24.04.2002
(21) Application number: 93916942.1
(22) Date of filing: 02.07.1993
(51) Int. Cl.: G01N 33/576

(54) **IMMUNOASSAYS FOR ANTI-HCV ANTIBODIES USING ANTIGENS WITH CONFORMATIONAL EPITOPES**
IMMUNOASSAYS FÜR ANTI-HCV-ANTIKÖRPER UNTER VERWENDUNG VON ANTIGENEN MIT KONFORMATIONSEPITOPEN
DOSAGES IMMUNOLOGIQUES POUR ANTICORPS DIRIGES CONTRE LE VIRUS DE L'HEPATITE C (HCV) A L'AIDE D'ANTIGENES COMPRENANT DES EPITOPES A CONFORMATION

(30) Priority: 07.07.1992 US 910759
(43) Date of publication of application: 26.04.1995
(73) Proprietor: CHIRON CORPORATION, Emeryville, California 94608-2916 (US)
(72) Inventor: CHIEN, David, Y., Alamo, CA 94507 (US)
(74) Representative: Hallybone, Huw George
(86) International application number: PCT/US1993/006309
(87) International publication number: WO 1994/001778

(56) References cited:
- EP-A- 0 388 232
- EP-A- 0 451 891
- EP-A- 0 468 657
- WO-A-91/15516
- WO-A-92/08734
- GB-A- 2 239 245
- US-A- 4 855 224
- BIOLOGICAL ABSTRACTS vol. 95, no. 12 , 15 June 1993, Philadelphia, PA, US; abstract no. 131505, SALLBERG, M.; U. RUDEN; B. WAHREN AND L.O. MAGNUS 'Antigenic regions within the hepatitis C virus envelope 1 and non-structural proteins: Identification of an IgG3-restricted recognition site within the envelope 1 protein'
- VIROLOGY vol. 188, no. 2, June 1992, ORLANDO FL US pages 819 - 830 RICHARD R. SPAETE ET AL. 'Characterization of the Hepatitis C Virus E2/NS1 Gene Product Expressed in Mammalian Cells' cited in the application
- Tremolada F. et al., Ann Intern Med. Feb,15,1991
- Ashley RI. et al., J Clin Microbiol., Apr. 1988, 26/4) P.662-7
- Gerberding JL. et al., Int Conf. AIDS, Jun 20-2, 1990, 6 (1) P275 (abstract no. Th.C.601).

## Description

### Technical Field

The invention relates to materials and methodologies for managing the spread of hepatitis C virus (HCV) infection. More specifically, it relates to polypeptides useful immunological reagents in the detection, prevention and treatment of HCV infections, as well as immunoassays and kits employing.

### Background

HCV was first identified and characterized as the primary cause of post transfusion non-A, non-B hepatitis (NANBH) by Houghton et al. In addition to providing substantial information concerning HCV, Houghton et al. and their collaborators have disclosed a number of general and specific immunological reagents and methods. See, e.g. Houghton et al., EPO Pub. No. 318,216; Houghton et al., EPO Pub. No. 388,232; Chao et al., Science (1989) 244: 359-362; Kuo et al., Science (1989) 244:362-364; Takeuchi et al., J. Gen. Virol. (1990) 71:3027-3033; Takeuchi et al., Gene (1990) 91:287-291; Takeuchi et al., Nucl. Acids Res. (1990) 18:4626; Miyamura et al. Proc. Natl. Acad. Sci. USA . (1990) 87: 983-987: Salto et al., Proc. Natl: Acad. Sci. USA (1990) 87: 6547-6549 Choo et al., Proc. Natl. Acad, Sci. USA-(1991) 88:2451-2455; Han et al., Proc. Natl. Acad. Sci. USA 88 1711-1715; Houghton et al., Hepatology (1991) 14:381-388; Weiner et al., Proc. Natl. Acad. Sci. USA (1992) 89: 3468-3472. These publications provide the art with an extensive background on HCV generally, as well as the manufacture and uses of HCV polypeptide immunological reagents: For brevity, therefore, the disclosure of these publications in particular are incorporated herein by reference.

Others have readily applied and extended the work of Houghton et al. See, e.g. Highfield et al., UK Pat App. 2,239,245 (The Welcome Foundation Ltd.): Wang, EPO Pub. No. 442,394 (United Biomedical Inc.); Leung et al., EPO Pub. No. 445,423 (Abbott Laboratories); Habits et al., EPO Pub. No. 451,891 (Akzo N.V.); Reyes et al., PCT Pub No. WO 91/15516 (Genelabs Inc.): Maki et al., EPO Pub; No. 468,657 (Tonen Corp.): and Kamada et al., EPO Pub. No. 469,348 (Shionogi Seiyaku K.K.). See also Matsuura et al., (1992) J. Virology 66:1425; Kato et al., Proc. Natl. Acad. Sci. USA (1990) 87: 9524-9528: Takamizawa et al., J. Virol, (1991) 65:1105-1113; Chiba et al., Proc. Natl. Acad. Sci. USA (1991) 88 4641-4645; Harada et al., J. Virol. (1990) 65: 3015-3021; Hijikata et al., Proc. Natl. Acad. Sci. USA (1991) 88: 5547-5551; Okamoto et al., Jpn. J. Exp. Med. (1990) 60: 167-177; Yuasa et al., J. Gen. Virol. (1991) 72: 2021-2024; Watanabe et al., Int. J. Cancer (1991) 48: 340-343.

Sensitive, specific methods for screening and identifying carriers of HCV and HCV-contaminated blood or blood products are an important advance in medicine. Post-transfusion hepatitis (PTH) occurs in approximately 10% of transfused patients, and HCV has accounted for up to 90% of these cases. The major problem in this disease is the frequent progression to chronic liver damage (25-55%) relative to other hepatitises, such as type B.

Patient care as well as the prevention of transmission of HCV by blood and blood products orby close personal contact require reliable diagnostic and prognostic tools, such as HCV polypeptides, to detect antibodies related to HCV. Such polypeptides are also useful as vaccines and immunotherapeutic therapeutic agents for the prevention and/or treatment of the disease. Since HCV is a relatively new agent, a continuing need exists to define additional immunological reagents that will allow further study of the clinical course of disease and the epidemiology of HCV in the population.

### Disclosure of the Invention

Applicants have carried out additional serological studies on HCV antigens, and have discovered that immunoassays utilizing HCV envelope antigens that maintain conformational epitopes are more effective for detecting anti-HCV antibodies than are those utilizing the same antigens with linear epitopes.

Accordingly, one aspect of this invention is an in vitro method for detecting antibodies which arise relatively early during the course of infection in a body component of a mammalian host to hepatitis C virus (HCV), comprising the steps of:
(a) contacting said body component suspected of containing antibodies to HCV with (1) an HCV antigen selected from an antigen encoded in the E1 domain (amino acids 192 to 383) of the HCV genome, the E2 domain (amino acids 384 to 800) of the HCV genome or aggregates thereof, wherein said HCV antigen is purified under non-denaturing conditions or (2) a standard control, under conditions that allow an immunological reaction to occur; and
(b) detecting the presence of immune complexes of said antibodies and said antigen, wherein the absence of said immune complexes containing the standard control and the presence of said immune complexes containing said HCV antigen indicate seroconversion to HCV.

In other embodiments, the antigens are encoded in the E1 and/or E2 domains, or are the E1 and/or E2 antigens. In some embodiments, the antigens may be expressed from Vaccinia vectors, or be expressed in CHO cells. In addition, in some embodiments of the invention, aggregates of said antigens are included.

### Brief Description of the Drawings

Figure 1 is a schematic indicating the putative domains of the HCV polyprotein.
Figure 2 is a schematic showing some of the features of the vector, pSC59.
Figures 3A, 3B and 3C show the percentage of Group I, II and III patients reactive In ELISA assays against individual HCV antigens.

### Detailed Description of the Invention

"HCV antigen" refers to a polypeptide or polypeptide analog (e.g., mimitopes) comprising an amino acid sequence (and/or amino acid analogs) defining at least one HCV epitope. Typically, the sequences defining the epitope correspond to the amino acid sequence of an HCV protein (either Identically orvla substitution of analogs of the native amino acid residue that do not destroy the epitope). In general, the epitope-defining sequence will be 5 or more amino acids in length, more typically 8 or more amino acids in length, and even more typically 10 or more amino acids In length. With respect to conformational epitopes, the length of the epitope defining sequence can be subject to wide variations, since it is believed that these epitopes are formed by the three-dimensional shape of the antigen (e.g., folding). Thus, the amino acids defining the epitope can be relatively few in number, but widely dispersed along the length of the molecule (or even on different molecules in the case of dimers, etc.), being brought into the correct epitope conformation via folding. The portions of the antigen between the residues defining the epitope may not be critical to the conformational structure of the epitope. For example, deletion or substitution of these intervening sequences may not affect the conformational epitope provided sequences critical to epitope conformation are maintained (e.g., cysteines involved in disulfide bonding, glycosylation sites, etc.).

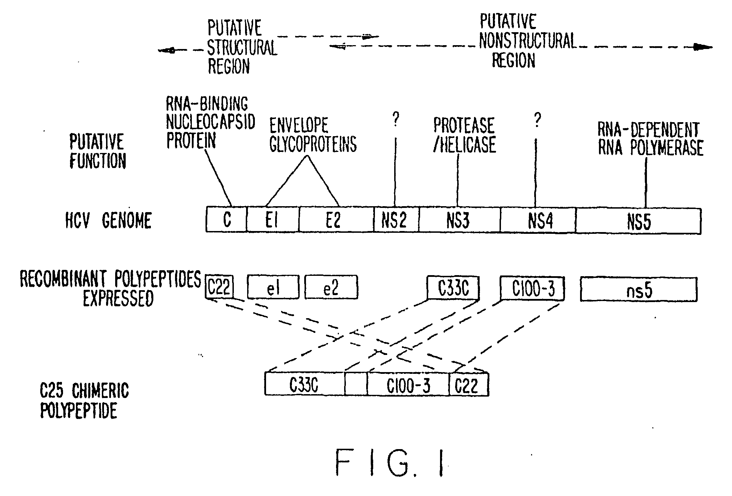

The HCV antigens of the present invention comprise conformational epitopes from the E1 and/or E2 (envelope) domains of HCV. The E1 domain, which is believed to correspond to the viral envelope protein, is currently estimated to span amino acids 192-383 of the HCV polyprotein (PCT Pub. No. WO91/15771, Figure 1). Upon expression in a CHO system (glycosylated), it is believed to have an approximate molecular weight of 35 Kd as determined via SDS-PAGE. The E2 protein, previously called NS1, is believed to span amino acids 384-800 of the polyprotein and to also be an envelope protein. Upon expression In a CHO system (glycosylated), it is believed to have an apparent gel molecular weight of about 72 Kd. It is understood that these protein endpoints are approximations (e.g., the carboxy terminal of E2 could lie somewhere in the 750-820 amino acid region). It is also understood that the prototype isolate HCV1 sequence in the aforementioned PCT application is cited for illustrative purposes only and that any HCV isolate (see, e.g., references cited In the "Background" section) is a suitable source of E1 and/or E2 sequence for the practice of the present invention.

The E1 and E2 antigens used in the present invention may be full-length viral proteins, substantially full-length versions thereof, or functional fragments thereof (e.g., fragments which are not missing sequence essential to the formation or retention of a conformational epitope). Furthermore, the HCV antigens of the present invention can also include other sequences that do not block or prevent the formation of the conformational epitope of interest. The presence or absence of a conformational epitope can be readily determined through screening the antigen of interest with an antibody (polyclonal serum or monoclonal to the conformational epitope) and comparing its reactivity to that of a denatured version of the antigen which retains only linear epitopes (if any). In such screening using polyclonal antibodies, it may be advantageous to adsorb the polyclonal serum first with the denatured antigen and see if it retains antibodies to the antigen of interest.

The HCV antigens of the present invention can be made by any convenient method that provides the conformational epitope of interest. For example, recombinant expression in mammalian or insect cells is a preferred method to provide secreted glycosylated E1 and/or E2 antigens in "native" conformation. However, it may also be possible, as it is known for proteins, to express the antigens in other recombinant hosts and renature the protein after recovery. It is also understood that chemical synthesis may also provide conformational antigen mimitopes that cross-react with the "native" antigen's conformational epitope.

Complexes of E1 and/or E2 (also called aggregates) containing more than one E1 or E2 monomer are also preferred antigens. E1:E1 dimers, E2:E2 dimers, and E1:E2 heterodimers are all antigens within the scope of this invention. Aggregates may also include larger forms, and may have molecular weights in excess of 800 kD.

"Fusion polypeptide" intends a polypeptide in which the HCV antigen(s) are part of a single continuous chain of amino acids, which chain does not occur in nature. The HCV antigens may be connected directly to each other by peptide bonds or be separated by intervening amino acid sequences. The fusion polypeptides may also contain amino acid sequences exogenous to HCV.

"Common solid matrix" intends a solid body to which the individual HCV antigens or the fusion polypeptide comprised of HCV antigens are bond covalently or by noncovalent mean such as hydrophobic adsorption.

"Body component" intends a fluid or tissue of a mammalian individual (e.g., an anthropoid, a human) that commonly contains antibodies produced by the individual. Such components are known in the art and include, without limitation, blood, plasma, serum, spinal fluid, lymph fluid, secretions of the respiratory, intestinal or genitourinary tracts, tears, saliva, milk, white blood cells, and myelomas. Body components include biological liquids. The term "biological liquid" refers to a fluid obtained from an organism. Some biological fluids are used as a source of other products, such as clotting factors (e.g., Factor VIII:C), serum albumin, growth hormone, and the like. In such cases, it is important that the source of biological fluid be free of contamination by virus such as HCV.

"Immunologically reactive" means that the antigen in question will react specifically with anti-HCV antibodies present in a body component from an HCV infected individual.

"Immune complex" intends the combination formed when an antibody binds to an epitope on an antigen.

"E1" as used herein refers to a protein or polypeptide expressed within the first 400 amino acids of an HCV polyprotein, sometimes referred to as the E or S protein. In its natural form it is a 35 kD glycoprotein which is found in strong association with membrane. In most natural HCV strains, the E1 protein is encoded in the viral polyprotein following the C (core) protein. The E1 protein extends from approximately amino acid (aa) 192 to about aa 383 of the full-length polyprotein. The term "E1" as used herein also includes analogs and truncated forms that are immunologically cross-reactive with natural E1.

"E2" as used herein refers to a protein or polypeptide expressed within the first 900 amino acids of an HCV polyprotein, sometimes referred to as the NS1 protein. In its natural form it is a 72 kD glycoprotein that is found in strong association with membrane. In most natural HCV strains, the E2 protein is encoded in the viral polyprotein following the E1 protein. The E2 protein extends from approximately aa 334 to about aa 820. The term "E2" as used herein also includes analogs and truncated forms that are immunologically cross-reactive with natural E2.

The term "aggregate" as used herein refers to a complex of E1 and/or E2 containing more than one E1 or E2 monomer. E1:E1 dimers. E2:E2 dimers, and E1 1:E2 heterodimers are all "aggregates" within the scope of this definition. Aggregates may also include larger forms, and may have molecular weights in excess of 800 kD.

The term "purified" as applied to proteins herein refers to a composition wherein the desired protein comprises at least 35 % of the total protein component in the composition. The desired protein preferably comprises at least 40%, more preferably at least about 50%, more preferably at least about 60%, still more preferably at least about 70%, even more preferably at least about 30%, even more preferably at least about 90%, and most preferably at least about 95% of the total protein component. The composition may contain other compounds such as carbohydrates, salts, lipids, solvents, and the like, without affecting the determination of the percentage purity as used herein. An "isolated" HCV protein intends an HCV protein composition that is at least 35 % pure.

"The term "isolated polypeptide" refers to a polypeptide which has been substantially free from other HCV viral components, particularly genomic HCV polynucleotide. A polypeptide composition is "substantially free" of another component if the weight of the polypeptide in the composition is at least 70% of the weight of the polypeptide and other component combined, more preferably at least about 89%, still more preferably about 90%, and most preferably 95% or greater.

### Modes for Carrying Out The Invention

The immunoassay methods of the invention utilize HCV antigens from the E1 and E2 domains and that maintain conformational epitopes recognized by antibodies in the sera of individuals infected with HCV. The antigens each have at least one conformational epitope that exists in the naturally occurring HCV particle or its infective product, as evidenced by the immunoreactivity of the antigen with antibodies in a body componentfrom an HCV infected individual, and a loss of the epitope's immunoreactivity upon denaturation of the antigen. The length of the antigen is sufficient to maintain an immunoreactive conformational epitope associated with that epitope. It is within the scope of the invention to have more than one conformational epitope on an antigen. Often the native envelope antigens used in the immunoassays may be almost full length, but may be truncated to, for example, increase solubility or to improve secretion (e.g., deletion of membrane binding domains). It is also within the scope of the invention to use either single antigens, or combinations or aggregates of antigens. Thus, the immunoassays may use E1 epitopes, E2 epitopes aggregates or combinations of native E1 and E2. It is also contemplated to be within the scope of the invention to use antigens with linear epitopes in combination with HCV envelope conformational epitopes.

Methods for detecting the presence of conformational epitopes are known within the art, and some are illustrated infra in the Examples.

Based upon the putative amino acids encoded in the nucleotide sequence of HCV1 and other evidence, possible protein domains of the encoded HCV polyprotein, as well as approximate boundaries are the following:

| Putative Domain | Approximate Boundary (amino acid nos.) |
|---|---|
| C (nucleocapsid protein) | 1-191 |
| E1 (virion envelope protein) | 192-383 |
| E2/NS1 (envelope?) | 384-800 |
| NS2 | 800-1050 |
| NS3 (protease?) | 1050-1650 |
| NS4 | 1651-2100 |
| NS5 (polymerase) | 2100-3011 (end) |

These domains are, however, tentative. For example, the E2-NS2 border is probably in the 750-810 region, and the NS3-NS4 border is about 1640-1650. There is also evidence that the 191 aa version of C is a precursor that is further processed (e.g., to about 170 aa in length), and that the NS2, NS4 and NS5 proteins are each further processed into two mature proteins. The relationship of the domains are illustrated in Figure 1.

Methods for preparing E1 and E2 antigens including those with native conformations are described in Spaete R., et al., Virology (1992) 188:819-830, and in WO92/08734, which are incorporated herein by reference. Generally, host cells are chosen that will allow the formation of native conformational epitopes within the expressed envelope proteins: these host cells may include, for example, animal cells, insect cells, yeast cells, and the like.

Eukaryotic hosts include yeast and mammalian cells in culture systems. Saccharomyces cerevisiae and Saccharomyces carlsbergensis are the most commonly used yeast hosts, and are convenient fungal hosts. Yeast compatible vectors carry markers which permit selection of successful transformants by conferring prototropy to auxotrophic mutants or resistance to heavy metals on wild-type strains. Yeast compatible vectors may employ the 2 micron origin of replication (Broach et al. (1983) Meth. Enz. 101:307.), the combination of CEN3 and ARS1 or other means for assuring replication, such as sequences which will result in incorporation of an appropriate fragment into the host cell genome. Control sequences for yeast vectors are known in the art and include promoters for the synthesis of glycolytic enzymes (Hess et al. (1968) J. Adv. Enzyme Reg 7:149; Holland et al. (1978) Biochemistry 17:4900), including the promoter for 3 phosphoglycerate kinase (Hitzeman (1980) J. Biol. Chem. 255:2073). Terminators may also be included, such as those derived from the enolase gene (Holland (1981) J. Biol. Chem. 256: 1385.). Particularly useful control systems are those which comprise the glyceraldehyde-3 phosphate dehydrogenase (GAPDH) promoter or alcohol dehydrogenase (ADH) regulatable promoter, terminators also derived from GAPDH, and if secretion is desired, leader sequence from yeast alpha factor. In addition, the transcriptional regulatory region and the transcriptional initiation region which are operably linked may be such that they are not naturally associated in the wild-type organism. These systems are described in detail in EPO 120,551, published October 3, 1984; EPO 116,201, published August 22, 1984; and EPO 164,556. published December 18. 1985, all of which are assigned to the herein assignee, and are hereby incorporated herein by reference.

Mammalian cell lines available as hosts for expression are known in the art and include many immortalized cell lines available from the American Type Culture Collection (ATCC), including HeLa cells, Chinese hamster ovary (CHO) cells, baby hamster kidney (BHK) cells, and a number of other cell lines. Suitable promoters for mammalian cells are also known in the art and include viral promoters such as that from Simian Virus 40 (SV40) (Piers (1978) Nature 273:113), Rous sarcoma virus (RSV), adenovirus (ADV), and bovine papilloma virus (BPV). Mammalian cells may also require terminator sequences and poly A addition sequences; enhancer sequences which increase expression may also be included, and sequences which cause amplification of the gene may also be desirable. These sequences are known in the art.

Vectors suitable for replication in mammalian cells are known in the art, and may include viral replicons, or sequences which insure integration of the appropriate sequences encoding NANBV epitopes into the host genome.

A vector which is used to express foreign DNA, and which may be used in vaccine preparation is Vaccinia virus. In this case the heterologous DNA is inserted into the Vaccinia genome. Techniques for the insertion of foreign DNA into the vaccinia virus genome are known in the art, and utilize, for example, homologous recombination. The insertion of the heterologous DNA is generally into a gene which is non-essential in nature, for example, the thymidine kinase gene (tk) which also provides a selectable marker. Plasmid vectors that greatly facilitate the construction of recombinant viruses have been described (see, for example, Mackett et al. (1984) J. Virol. 49:857., Chakrabarti et al. (1985) Mol. Cell Biol. 5:3403; Moss (1987) in GENE TRANSFER VECTORS FOR MAMMALIAN CELLS (Miller and Calos, eds., Cold Spring Harbor Laboratory, Cold Spring Harbor, N.Y.), p. 10.). Expression of the HCV polypeptide then occurs in cells or individuals which are immunized with the live recombinant vaccinia virus.

The segment of HCV CDNA encoding the desired sequence is inserted into a Vaccinia vector. The polypeptide encoding sequence may be attached to a leader sequence. The leader sequence may be that for tissue plasminogen activator (TPA), or from another source, e.g., that for beta-globin. The heterologous polynucleotide may be inserted into a vaccinia vector which is a modified version of pSC11, due to the addition of a polylinker sequence which contains a cloning site.

In order to detect whether or not the HCV polypeptide is expressed from the vaccinia vector, BSC 1 cells may be infected with the recombinant vector and grown on microscope slides under conditions which allow expression. The cells may then be acetone-fixed, and immunofluorescence assays performed using serum which is known to contain anti-HCV antibodies to a polypeptide(s) encoded in the region of the HCV genome from which the HCV segment in the recombinant expression vector was derived.

Other systems for expression of eukaryotic or viral genomes include insect cells and vectors suitable for use in these cells. These systems are known in the art, and include, for example, insect expression transfer vectors derived from the baculovirus Autographa californica nuclear polyhedrosis virus (AcNPV), which is a helper-independent, viral expression vector. Expression vectors derived from this system usually use the strong viral polyhedrin gene promoter to drive expression of heterologous genes. Currently the most commonly used transfer vector for introducing foreign genes into AcNPV is pAc373. Many other vectors, known to those of skill in the art, have also been designed for improved expression. These include, for example, pVL985 (which alters the polyhedrin start codon from ATG to ATT, and which introduces a BamHI cloning site 32 basepairs downstream from the ATT; See Luckow and Summers (1989) Virology 17:31). Good expression of nonfused foreign proteins usually requires foreign genes that ideally have a short leader sequence containing suitable translation initiation signals preceding an ATG start signal. The plasmid also contains the polyhedrin polyadenylation signal and the ampicillin-resistance (amp) gene and origin of replication for selection and propagation in E. coli.

Methods for the introduction of heterologous DNA into the desired site in the baculovirus virus are known in the art. (See Summer and Smith. Texas Agricultural Experiment Station Bulletin No. 1555: Ju et al. (1987) : Smith et al. (1933) Mol. & Cell Biol. 3:2156-2165: and Luckow and Summers (1989) Virology 17:31). For example the insertion can be into a gene such as the polyhedrin gene, by homologous recombination; insertion can also be into a restriction enzyme site engineered into the desired baculovirus gene. The inserted sequences may be those which encode all or varying segments of the polyprotein or other orfs which encode viral polypeptides.

The signals for posttranslational modifications such as signal peptide cleavage proteolytic cleavage, and and phosphorylation, appear to be recognized by insect cells. The signals required for secretion and nuclear accumulation also appear to be conserved between the invertebrate cells and vertebrate cells. Examples of the signal sequences from vertebrate cells which are effective in invertebrate cells are known in the art, for example, the human interleukin-2 signal (IL-2₅) which is a signal for transport out of the cell, is recognized and properly removed in insect cells.

### Immunoassay Formats

The HCV E1 and E2 antigens of the present invention may be employed in virtually any assay format that employs a known antigen to detect antibodies. Of course, formats that denature the HCV conformational epitope of interest should be avoided or modified.A common feature of all of these assays is that the antigen is contacted with the body component suspected of containing HCV antibodies under conditions that permit the antigen to bind to any such antibody present in the component. Such conditions will typically be physiologic termperature, Ph and ionic strength using an excess of antigen. The incubation of the antigen with the specimen is followed by detection of immune complexes comprised of the antigen.

Design of the immunoassays is subject to a great deal of variation, and many formats are known in the art. Protocols may, for example, use solid supports, or immunoprecipitation. Most assays involve the use of labeled antibody or polypeptide; the labels may be, for example, enzymatic, fluorescent, chemiluminescent, radioactive, or dye molecules. Assays which amplify the signals from the immune complex are also known; examples of which are assays which utilize biotin and avidin, and enzyme-labeled and mediated immunoassays, such as ELISA assays.

The immunoassay may be, without limitation, in a heterogeneous or in a homogeneous format, and of a standard or competitive type. In a heterogeneous format, the polypeptide is typically bound to a solid matrix or support to facilitate separation of the sample from the polypeptide after incubation. Examples of solid supports that can be used are nitrocellulose (e.g., in membrane or microtiter well form), polyvinyl chloride (e.g., in sheets or microtiter wells), polystyrene latex (e.g., in beads or microtiter plates, polyvinylidine fluoride (known as Immunlon™), diazotized paper, nylon membranes, activated beads, and Protein A beads. For example, Dynatech Immunlon™ 1 or Immunlon™ 2 microtiter plates or 6.4 mm (0.25 inch) polystyrene beads (Precision Plastic Ball) can be used in the heterogeneous format. The solid support containing the antigenic polypeptides is typically washed after separating it from the test sample, and prior to detection of bound antibodies. Both standard and competitive formats are known in the art.

In a homogenous format, the test sample is incubated with the combination of antigens in solution. For example, it may be under conditions that will precipitate any antigen-antibody complexes which are formed. Both standard and competitive formats for these assays are known in the art.

In a standard format, the amount of HCV antibodies in the antibody-antigen complexes is directly monitored. This may be accomplished by determining whether labeled anti-xenogeneic (e.g., anti-human) antibodies which recognize an epitope on anti-HCV antibodies will bind due to complex formation. In a competitive format, the amount of HCV antibodies in the sample is deduced by monitoring the competitive effect on the binding of a known amount of labeled antibody (or other competing ligand) in the complex.

Complexes formed comprising anti-HCV antibody (or in the case of competitive assays, the amount of competing antibody) are detected by any of a number of known techniques, depending on the format. For example, unlabeled HCV antibodies in the complex may be detected using a conjugate of antixenogeneic Ig complexed with a label, (e.g., an enzyme label).

In an immunoprecipitation or agglutination assay format the reaction between the HCV antigens and the antibody forms a network that precipitates from the solution or suspension and forms a visible layer or film of precipitate. If no anti-HCV antibody is present in the test specimen, no visible precipitate is formed.

There currently exist three specific types of particle agglutination (PA) assays. These assays are used for the detection of antibodies to various antigens when coated to a support. One type of this assay is the hemagglutination assay using red blood cells (RBCs) that are sensitized by passively adsorbing antigen (or antibody) to the RBC. The addition of specific antigen antibodies present in the body component, if any, causes the RBCs coated with the purified antigen to agglutinate.

To eliminate potential non-specific reactions in the hemagglutination assay, two artificial carriers may be used instead of RBC in the PA. The most common of these are latex particles. However, gelatin particles may also be used. The assays utilizing either of these carriers are based on passive agglutination of the particles coated with purified antigens.

The HCV E1 and E2 antigens comprised of conformational epitopes will typically be packaged in the form of a kit for use in these immunoassays. The kit will normally contain in separate containers the native HCV antigen, control antibody formulations (positive and/or negative), labeled antibody when the assay format requires same and signal generating reagents (e.g.. enzyme substrate) if the label does not generate a signal directly. The native HCV antigen may be already bound to a solid matrix or separate with reagents for binding it to the matrix. Instructions (e.g., written, tape. CD-ROM etc.) for carrying out the assay usually will be included in the kit.

Immunoassays that utilize the native HCV antigen are useful in screening blood for the preparation of a supply from which potentially infective HCV is lacking. The method for the preparation of the blood supply comprises the following steps. Reacting a body component, preferably blood or a blood component, from the individual donating blood with native HCV E1 or native HCV E2 antigen to allow an immunological reaction between HCV antibodies, if any, and the HCV antigen. Detecting whether anti-HCV antibody - HCV antigen complexes are formed as a result of the reacting. Blood contributed to the blood supply is from donors that do not exhibit antibodies to the native HCV antigens, E1 or E2.

In cases of a positive reactivity to the HCV antigen, it is preferable to repeat the immunoassay to lessen the possibility of false positives. For example, in the large scale screening of blood for the production of blood products (e.g., blood transfusion, plasma, Factor VIII, immunoglobulin, etc.), "screening" tests are typically formatted to increase sensitivity (to insure no contaminated blood passes) at the expense of specificity; i.e., the false-positive rate is increased. Thus, it is typical to only defer for further testing those donors who are "repeatedly reactive"; i.e., positive in two or more runs of the immunoassay on the donated sample.

The following examples are intended to illustrate the invention and are not intended to limit the invention in any manner.

### Example 1

### Construction of PSC59 Poly

The HCV sequence used for the construction was isolated from plasmid pC5P-1 as a Stul partial/Bg1 II fragment. This fragment extends from the first methionine of HCV-1 polyprotein to aspartic acid at position 966. The domains included are the nucleocapsid, C, both putative envelope glycoproteins, E1 and E2, and a truncated form of NS2, respectively. In addition, the fragment also contains about 60 bp corresponding to that portion of the 5'-untranslated region of the HCV genome.

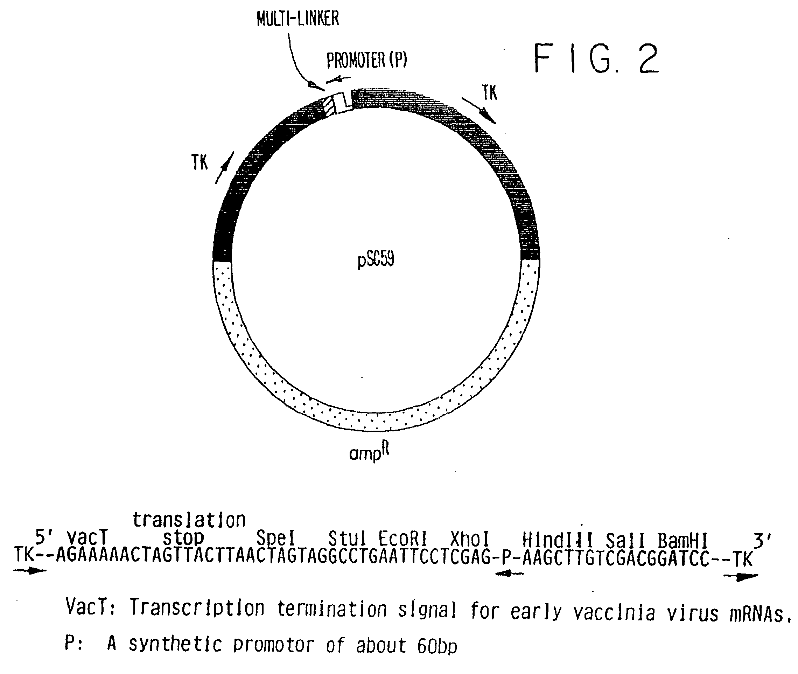

The fragment was treated with Klenow polymerase to create blunt ends, and then cloned into the Stul site of a vaccinia vector, PSC59 (obtained from Dr. B. Moss at the National Institutes of Health, Bethesda, Md). The vector is shown in Figure 2. As a result of the ligation into the polylinker sequence of the vector, the C'-terminus of the NS2 region contains an additional Pro-Tyr sequence.

### Example 2

### Preparation of Stocks of Vaccinia Virus Encoding the HCV Polyprotein Fragment Including E1 and E2

The screening for recombinant Vaccinia virus was carried out essentially as described by Mackett et al. in DNA Cloning, Vol. II (Ed. D.M. Glover, IRL Press, Oxford, England, 1985, pp. 191-211. More specifically, a confluent monolayer (6 cm dish) of African green monkey kidney cells, BSC40, was infected with wild type Vaccinia virus (WR strain) at a multiplicity of infection (MOI) of 0.05. After a 2 hour incubation at 37°C, the cells were transfected with 25 µg of PSC59 poly DNA using the calcium phosphate method. After 4 hours of incubation, the medium was changed to normal medium, and the cells were incubated for an additional 48 hours at 37°C. The cells were recovered by scraping them from the dish, and the virus was released by 3 cycles of freezing-thawing, and the released virus in the cell lysate were stored at -80°C.

In order to screen for recombinant virus, a confluent monolayer of human 143 TK cells were infected for 2 hours with the cell lysate in 10-fold serial dilutions. After removal of the inoculum, 1 % agarose in serum medium containing 25 µg/ml 5-bromodeoxyuridine was added, and the cells were incubated 72 hours at 37°C. Plaques were visualized by overlaying the cell layer with 1 % agarose plus 0.01% neutral red, and incubating the cells overnight at 37°C. The agarose overlay was then carefully removed, and the cell layer was blotted with a master nitrocellulose filter (Schleir and Schuell, BA85, 0.45µm). A replica plate of the master filter was made, and probed with a ³²P-labeled hybridization probe to the HCV sequence. Positive plaques were isolated from the master filter, placed in 0.5 ml serum-free medium, and sonicated twice for 30 seconds. The screening process was repeated twice to plaque purify the virus.

In order to propagate the recombinant Vaccinia virus, ten dishes (150 cm²) of BSC40 cells were infected with the viral stock at a MOI of 0.5. The infection was carried out for 2 hours at 37° C, and the viral stock replaced with fresh medium. After 72 hours the cells were harvested, suspended in 10 Mm Tris HCI. Ph 9.0. and homogenized in a Wheaton dounce tissue grinder. Cell debris was removed by centrifugation, the supernatants were trypsinized and sonicated, and aliquots of the viral suspensions were stored at -80°C.

### Example 3

### Production of E1/E2 Antigens

One liter of Hela S3 spinner cells were grown in a spinner flask to a density of 10⁶ cells per ml. The cells were infected with the recombinant Vaccinia virus encoding the HCV polyprotein fragment using a MOl of 1.0, incubated overnight, harvested, and stored as a cell pellet at -80°C.

The E1/E2 expression product was purified by lysing the cells in hypotonic buffer, followed by extraction in a buffer containing nonionic detergent. The cellularextractwas chromatographed through a lectin (GNA) agarose column. The desired proteins were eluted from the column with methyl-α-D-mannopyranoside (Sigma Corp.). The eluted fractions were monitored for E1 and E2 by Western blots using a specific antiserum raised against E1 or E2. The fractions containing the antigens were pooled and concentrated on a S-Sepharose column (Pharmacia). The purity of the final product was about 70%.

### Example 4

### Use of E1/E2 For Immunoassays

### Patients

Serum samples were obtained from randomly selected paid plasma donors collected from Eastern, Western. Midwestern and Southern States of the United States of America purchased from Uniglobe Research Corporation (Reseda, ICA). Serum samples provided by H. Tong (Huntington Memorial Hospital at Pasadena, California), were obtained from a group of 38 patients of transfusion associated chronic NANBH and another group of 39 patients of IV drug users with chronic NANBH. These 77 patients were free of other liver diseases, were anti-HAV and anti-Hbsag non-reactive, and anti-nuclear antibodies (ANA) were normal. Three transfusion associated HCV seroconversion panels were obtained from H. Alter (the National Institutes of Health, Bethesda, Maryland) and Serologicals Incorporation at Clarkston, Georgia. The acute resolved HCV samples were provided by the Max von Petlenkofer-Institute, University of Munich, Munich, Germany. The 19 cases of acute resolving NANBH were diagnosed based on liver biopsy and normal alanine amino transferase (ALT) levels during long term follow-up studies (2-11 years).

### Expression and Purification of Recombinant HCV Antigens

The C22 (119aa), E1 (130aa), E2 (251aa), NS5 (942aa) and chimeric C25 (also called c200/c22)(858aa) antigens were expressed as internal antigens within yeast, *S. cerevisiae,* as C-terminal fusions with human superoxide dismutase (SOD) using methods described previously by Kuo, G. et al., Science (1989) 244:362-364, and Cousens L., et al., Gene (1987) 61:265-272. The C33C antigen (266aa) was expressed as an internal SOD fusion polypeptide in *E. coli* using methods described earlier for the synthesis of the 5-1-1 antigen (see PCT WO89/046699; EPO Pub. No. 318,216; and Houghton et al., Science 244:359 (1989), which are incorporated herein by reference). Following cell breakage and centrifugation, the insoluble SOD fusion polypeptides were extracted from the cell pellets using either 5 M urea or 1 % SDS and purified using either gel filtration or a combination of ion-exchange chromatography (Q- and S-sepharose) and gel filtration chromatography (Sephacryl S-300 HR).

The HCV native E1 and E2 antigen [also called rVV (e1 & e2)] was purified from the endoplasmic reticulum of recombinant Vaccinia virus (rvv)-infected cells that contain the full length HCV E1 and E2 genes. Purification was accomplished by affinity chromatography, followed by ion exchange chromatography under non-denaturing conditions. These methods were as described in WO92/08734, which is incorporated herein by reference.

The native HCV E2 antigen, CHO-e2, was prepared essentially according to Spaete et al., Virology (1992) 188:819-830. More specifically, the mammalian CHO cell line producing CHO-e2 antigen was constructed from a plasmid containing an HCV-1 sequence encoding Ala383 to Glu661. The plasmid was then transfected into CHO cells to generate a stable line expressing full length e2 (also called e2/ns1) antigen. Clones exhibiting high expression were selected and expanded in roller bottles by growth in DME/H21 with 10% dialyzed fetal calf serum and the usual supplements plus 1.6 µM Methotrexate. The culture medium supernatant was harvested, and used for the purification of the CHO-e2 antigen. The purification scheme included affinity and ion exchangechromatography under non-denaturing conditions.

In order to perturb the native e2 putative conformational epitopes, denatured CHO-e2 was prepared by addition of DL-ditbiothreitol (DTT) to a final concentration of 10 Mm. 0.2% sodium dodecyl sulfate (SDS), and boiled at 100°C for 5 minutes. All purified recombinant HCV antigens were judged to be at least 90% pure by SDS polyacrylamide gel analysis and siaining with Coomassie blue.

### ELISA assays

The RVV(e1 & e2). CHO-e2 and denatured CHO-e2 antigens were diluted to optimal concentration in phosphate buffered saline (PBS) (pH7.4) and coated on Immulon™ 1 plates (Dynatech. Chantilly, FA). The ELISA assays were performed as follows. Test specimens on the plate were diluted 40-fold in sample diluent, and incubated for 1 hour at 37°C. then washed with wash buffer. Polyclonal goat anti-human IgG (H+L) antibody conjugated to horseradish peroxidase (HRP0) was added to each well. The plates were incubated for 1 hr at 37°C, and then washed, o-phenylenediamine 2 HCl (OPD) and hydrogen peroxide were added for HRP color development. The results were read using a plate reader at 492/620nm. The ELISA cutoff O.D. values for antigen RVV(e1 & e2), SOD, C25, C22, C33C and ns-5 was 0.4 plus the O.D. from the mean of the negative control. The cutoff values of CHO-e2 and denatured CHO-e2 were 0.3 O.D. plus the O.D. from the corresponding antigen negative control. The cutoff value of anti-C100-3 was 0.45 O.D. plus its mean negative control O.D..

### Immunoassay results

The results of the ELISA assays using the antigens described above to detect anti-HCV antibodies in blood components from United States patient groups are shown in Table 1.

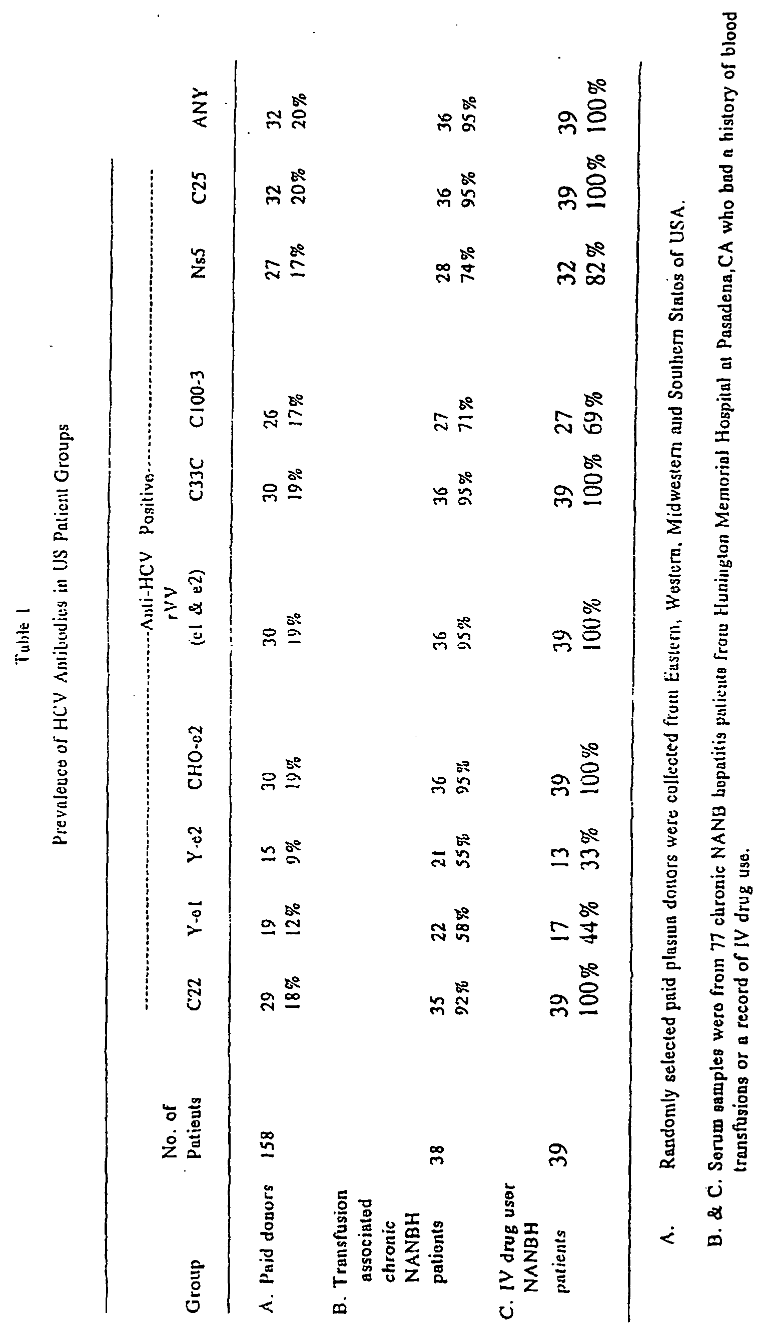

The results in Table 1 illustrate that the yeast produced HCV envelope antigens e1 and e2 are much less immunoreactive than the recombinant antigens produced in Vaccinia virus, RVV(e1 & e2), and in CHO cells, CHO-e2. In each group examined, the reactivity of the yeast produced antigens, e1 and e2, were only 2/3 or less of that of the antigens produced in the animal cells. It is noteworthy that the yeast e1 and e2 antigens were produced in yeast cells and purified under denaturing conditions. Thus, it appears that only linear epitopes exist in these antigens. In contrast, the native HCV antigens, RVV(e1 & e2) and CHO-e2 were isolated from the host animal cells under non-denaturing conditions, allowing the existence of native conformational epitopes.

The recombinant native HCV antigens expressed from the E1 and E2 genes in cells appeared to be as immunoreactive as C33C, and probably C25, and more immunoreactive than any of the other HCV antigens examined. C₂₅ is a chimeric polyprotein comprised of portions of the NS₃, NS₄ and C polypeptides, fused as shown in Figure 1.

The role of conformational epitopes in stimulation of host immune system early response was evaluated by immunoassay of HCV seroconversion panels. The results in Table 2 show the immunoreactivity of the native HCV envelope antigens, RVV(e1 & e2) and CHO-e2, with the samples in three seroconversion panels. The samples in the panels each represent sequential bleedings from an individual with post-transfusion NANBH. As seen from the results in Table 2, the native HCV envelope antigens, i.e., those with natural conformational epitopes, detect antibodies which arise relatively early during the course of infection, as compared to those detected by the other HCV antigens tested. Thus, these antigens may be of particular use in immunoassays for the diagnosis of HCV infection.

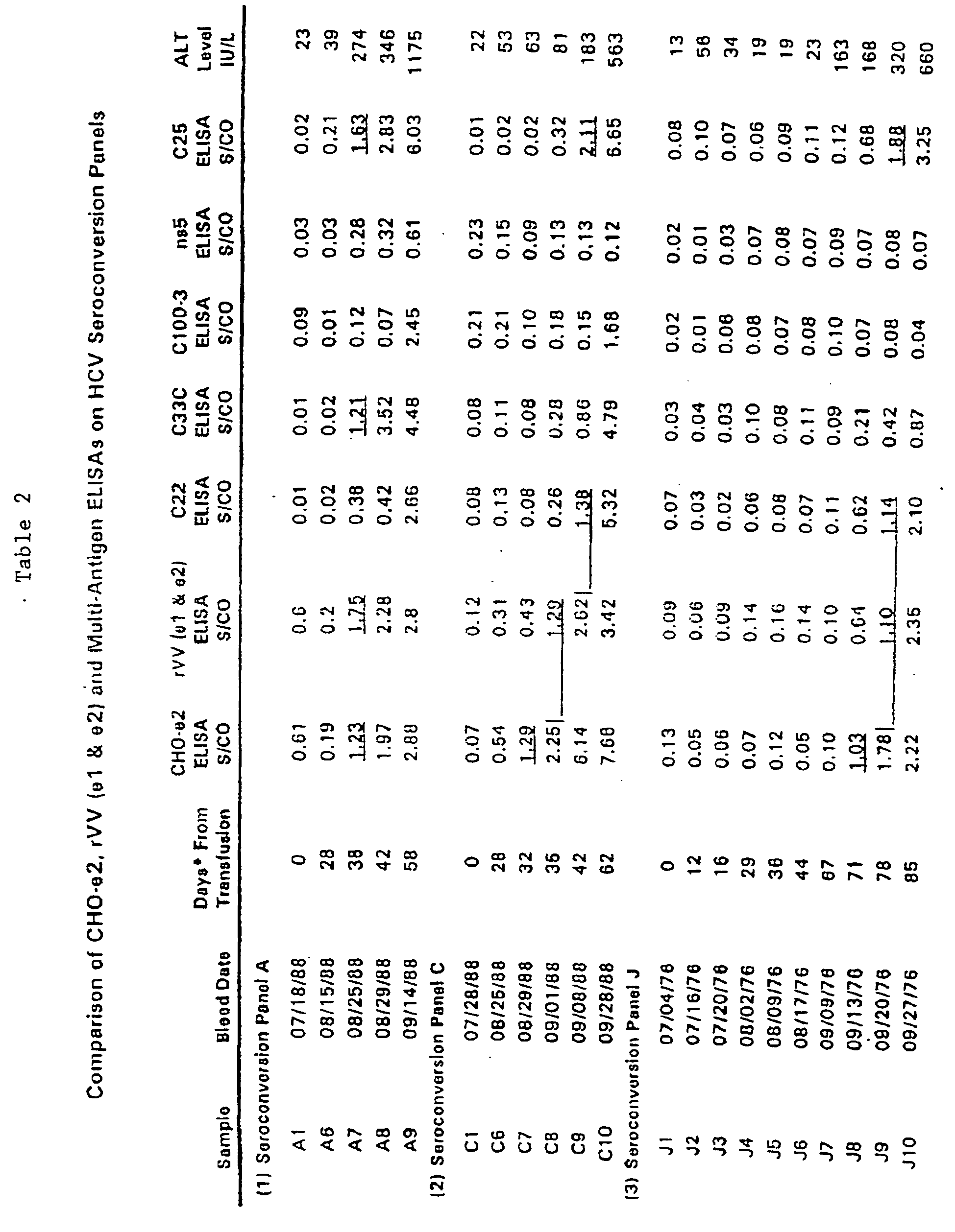

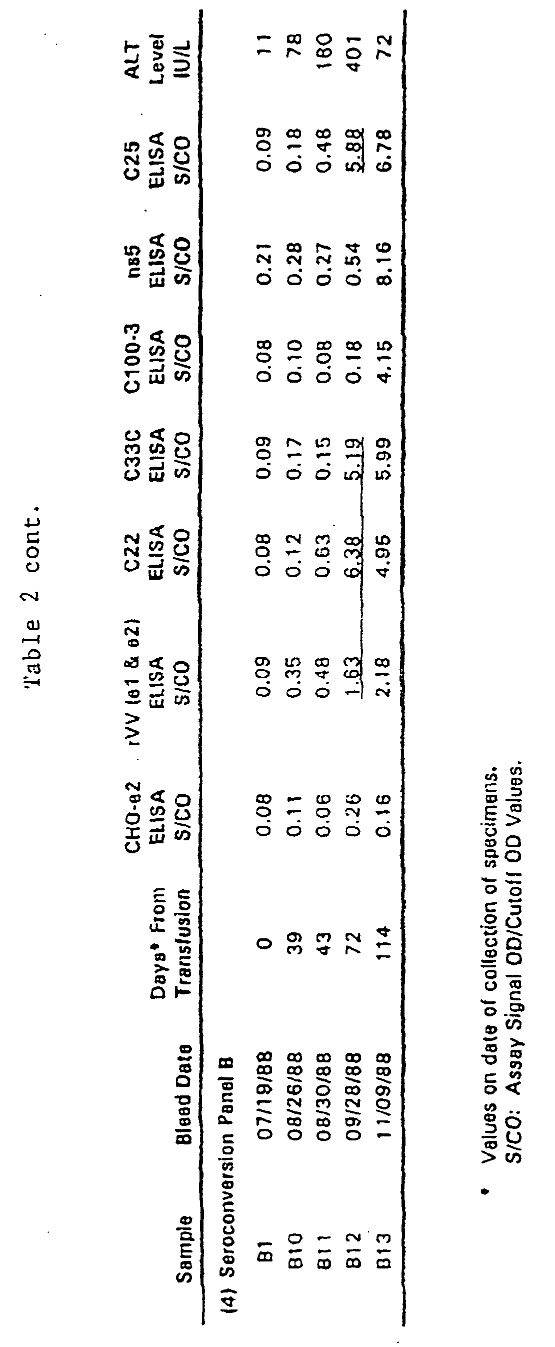

The results in Table 3 show a comparison of native HCV antigens expressed from the E1 and E2 genes, with the comparable denatured antigens. In the Table, immunoreactivity with serum from patients with acute resolving NANBH was followed with time, and compared to that from patients with chronic NANBH. As seen from Table 3, the native HCV envelope antigens that contain both conformational and linear epitopes, RVV(e1 & e2) and CHO-e2 are more immunoreactive than the antigens containing only linear epitopes, e1 and e2 (produced in yeast), and denatured CHO-e2. The overall observation is that the immunoreactivity of native HCV envelope antigens is increased between 8 to 9 fold with acute resolving patients, and about 2 fold with chronic NANBH patients. Thus, it appears that the majority of the immunologic response in patients with the resolving form of NANBH is to conformational epitopes, while that in the acute form is approximately evenly divided to conformational and linear epitopes.

These studies provide the first evidence that conformational epitopes may be involved in the host immunologic response to HCV.

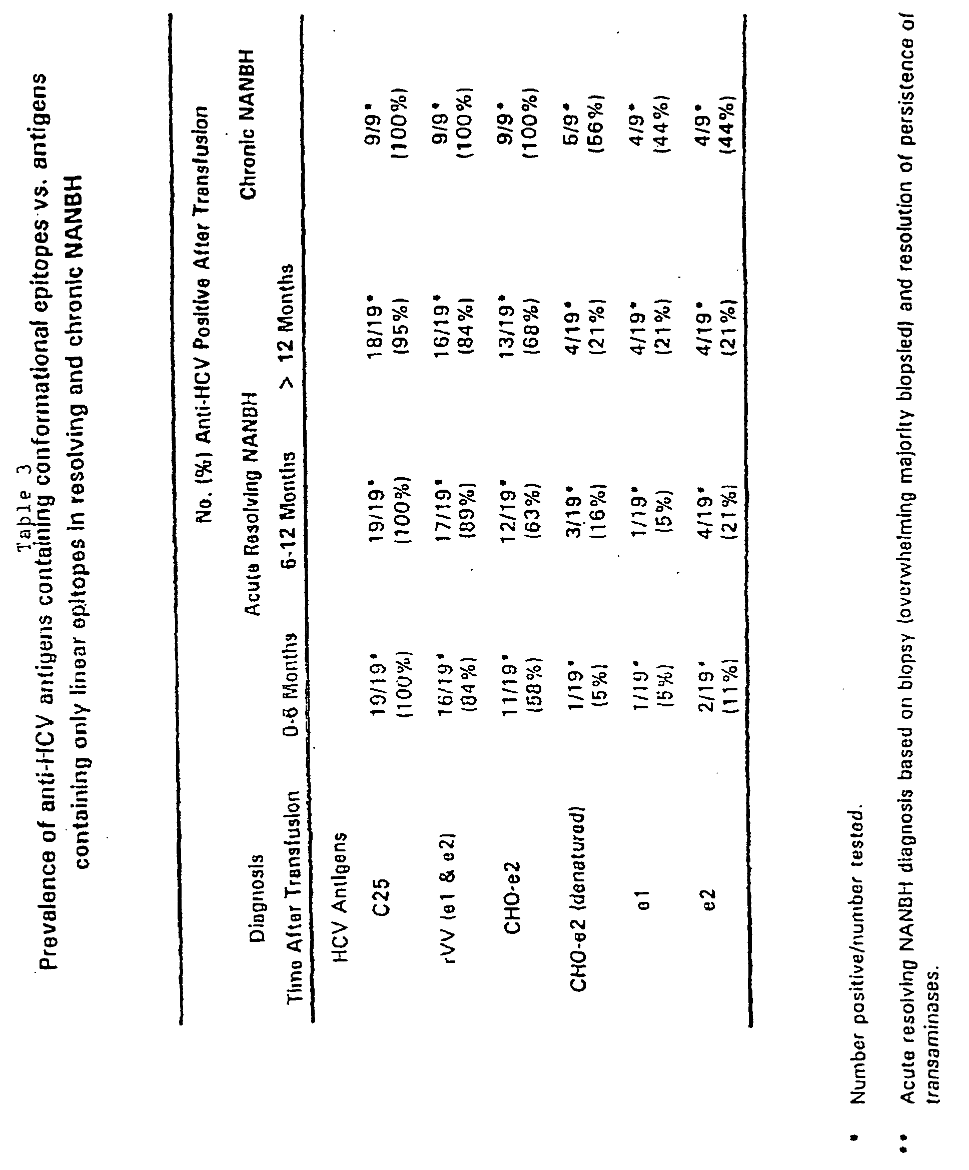

### Example 5

### E1/E2 Immunoassay: Comparison of Immunocompetent and Immunosuppressed Patients

### Patients

Three groups of patients were studied. All the patients were negative for antibody to human immunodeficiency virus (HIV). Group I consisted of 20 immunocompetent patients with transfusion related chronic HCV infection, who were otherwise healthy. All the patients were positive for anti-HCV and HCV RNA. Group II included 15 patients on maintenance hemodialysis. Eleven patients were anti-HCV positive at presentation, one received renal transplantation during follow-up. Four patients became anti-HCV positive during follow-up. Group III comprised 17 renal transplant recipients. Seven patients were anti-HCV and HCV RNA positive at presentation. Seven became anti-HCV positive during follow-up, two of whom were initially HCV RNA alone positive. Two patients were HCV RNA alone positive at presentation, both remained HCV RNA positive but anti-HCV negative during follow-up. One patient became positive for HCV RNA only during follow-up.

### Sera

Sera were prospectively collected between July 1989 and April 1992, and stored in aliquots at -70°C prior to testing. Two to four samples collected at intervals of 6-12 months were tested for patients in Group I. Two samples collected at an interval of 1.5 to 2 years were tested for all patients in Groups II and III; additional samples in the intervening period, and follow-up samples were also tested for patients who had changes in anti-HCV and/or HCV RNA status in the second sample.

### ELISA Assay

The HCV antigens were prepared as in Example 4 and used in ELISA assay as described in Example 4. In the N e1 & e2 assay, false positivity due to binding to vaccinia protein was reduced by (i) addition of vaccinia cell lysate to sample diluent to serve as competitive antigen, and (ii) use of vaccinia reactive samples as negative controls.

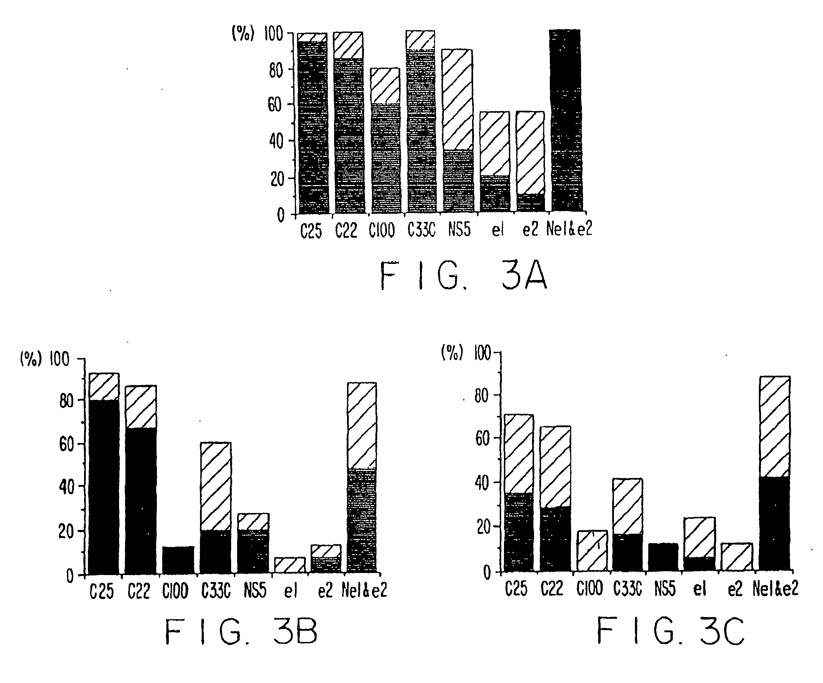

The results are shown in Figures 3A-3C. Only 55% of Group I immunocompetent patients reacted against e1 or e2 antigens expressed in yeast; but all patients reacted against native, glycosylated e1 & e2 antigens expressed in vaccinia virus. As in immunocompetent patients, most Group II hemodialysis patients were reactive to C25, C22, and N e1 & e2 antigens. A significantly lower proportion of patients reacted with C100-3. C33C. NS-5. e1 and e2. When compared to the immunocompetent patients, significantly less Group III renal transplant recipients reacted against all HCV antigens tested.

All the immunocompetent patients reacted to C25. C22, and C33C. 90% reacted to NS-5 and 80% to C100-3. Only 55% reacted against yeast derived e 1 & e2 antigens, but all reacted against vaccinia expressed N e1 & e2 antigens indicating that the envelope epitopes are conformational and glycosylated. 65 % - 90% of dialysis and renal transplant patients reacted to C25, C22 and N e1 & e2. but only 12%-60% reacted to C100-3, C33C and NS-5. Diminution or loss of reactivity to hepatitis C virus antigens were observed following renal and bone marrow transplantation, with with C25. and N e1 & e2 being less affected.

This suggests that e1 & e2 epitopes are conformational, and reactivity to these epitopes are minimally affected by immunosuppression relative to other HCV antigens.

## Claims

1. An in vitro method for detecting antibodies which arise relatively early during the course of infection in a body component of a mammalian host to hepatitis C virus (HCV), comprising the steps of:
(a) contacting said body component suspected of containing antibodies to HCV with (1) an HCV antigen selected from an antigen encoded in the E1 domain of an HCV genome, the E2 domain of an HCV genome or aggregates thereof, wherein said HCV antigen is purified under non-denaturing conditions or (2) a standard control, under conditions that allow an immunological reaction to occur; and
(b) detecting the presence of immune complexes of said antibodies and said antigen, wherein the absence of said immune complexes containing the standard control and the presence of said immune complexes containing said HCV antigen indicate seroconversion to HCV.

2. The method of claim 1 wherein said HCV antigen is encoded within the E1 domain of an HCV genome.

3. The method of claims 1 or 2 wherein said HCV antigen is E1.

4. The method of claim 1 wherein said HCV antigen is encoded within the E2 domain of an HCV genome.

5. The method of claims 1 or 4 wherein said HCV antigen is E2.

6. The method of claim 1 wherein said HCV antigen is an E1/E2 aggregate.

7. The method of claim 1 wherein said HCV antigen is an E1/E1 aggregate.

8. The method of claim 1 wherein said HCV antigen is an E2/E2 aggregate.

9. The method of any one of claims 1 to 8 wherein said HCV antigen or aggregate is expressed from recombinant vaccinia virus.

10. The method of any one of claims 1 to 8 wherein said HCV antigen or aggregate is expressed in CHO cells.

## Patentansprüche

1. In vitro-Verfahren zum Nachweis einer frühen Serokonversion in einer Körperkomponente eines Säugerwirts auf Hepatitis C-Virus (HCV), umfassend die Schritte:
(a) Inkontaktbringen der Körperkomponente, von der vermutet wird, dass sie Antikörper gegen HCV enthält, mit (1) einem HCV-Antigen, das aus einem Antigen, das in der E1-Domäne eines HCV-Genoms, das in der E2-Domäne eines HCV-Genoms oder das in Aggregaten davon codiert wird, ausgewählt wird, wobei das HCV-Antigen unter nicht-denaturierenden Bedingungen gereinigt wird, oder (2) einer Standardkontrolle unter Bedingungen, die es zulassen, dass eine immunologische Reaktion auftritt; und
(b) Nachweisen des Vorliegens von Immunkomplexen aus diesen Antikörpern und dem Antigen, wobei das Fehlen der Immunkomplexe, die die Standardkontrolle enthalten, und das Vorliegen der Immunkomplexe, die das HCV-Antigen enthalten, eine Serokonversion auf HCV anzeigen.

2. Verfahren nach Anspruch 1, wobei das HCV-Antigen in der E1-Domäne eines HCV-Genoms codiert wird.

3. Verfahren nach Anspruch 1 oder 2, wobei das HCV-Antigen E1 ist.

4. Verfahren nach Anspruch 1, wobei das HCV-Antigen in der E2-Domäne eines HCV-Genoms codiert wird.

5. Verfahren nach Anspruch 1 oder 4, wobei das HCV-Antigen E2 ist.

6. Verfahren nach Anspruch 1, wobei das HCV-Antigen ein E1/E2-Aggregat ist.

7. Verfahren nach Anspruch 1, wobei das HCV-Antigen ein E1/E1-Aggregat ist.

8. Verfahren nach Anspruch 1, wobei das HCV-Antigen ein E2/E2-Aggregat ist.

9. Verfahren nach einem der Ansprüche 1 bis 8, wobei das HCV-Antigen oder das Aggregat von rekombinantem Vaccinia-Virus exprimiert wird.

10. Verfahren nach einem der Ansprüche 1 bis 8, wobei das HCV-Antigen oder das Aggregat in CHO-Zellen exprimiert wird.

## Revendications

1. Méthode in vitro pour la détection d'anticorps qui apparaissent de manière relativement précoce pendant une infection dans un composant corporel d'un hôte mammifère au virus de l'hépatite C (HCV), comprenant les étapes consistant :
(a) à mettre en contact ledit composant corporel suspecté de contenir des anticorps contre le HCV avec (1) un antigène de HCV choisi parmi un antigène codé dans le domaine E1 d'un génome de HCV, le domaine E2 d'un génome de HCV ou des agrégats de ceux-ci, ledit antigène de HCV étant purifié en conditions non-dénaturantes ou (2) un témoin standard, dans des conditions qui permettent qu'une réaction immunologique ait lieu, et
(b) à détecter la présence de complexes immuns desdits anticorps et dudit antigène, où l'absence desdits complexes immuns contenant le témoin standard et la présence desdits complexes immuns contenant ledit antigène de HCV indiquent une séroconversion au HCV.

2. Méthode de la revendication 1, dans laquelle ledit antigène de HCV est codé dans le domaine E1 d'un génome de HCV.

3. Méthode de la revendication 1 ou 2, dans laquelle ledit antigène de HCV est E1.

4. Méthode de la revendication 1, dans laquelle ledit antigène de HCV est codé dans le domaine E2 d'un génome de HCV.

5. Méthode de la revendication 1 ou 4, dans laquelle ledit antigène de HCV est E2.

6. Méthode de la revendication 1, dans laquelle ledit antigène de HCV est un agrégat E1/E2.

7. Méthode de la revendication 1, dans laquelle ledit antigène de HCV est un agrégat E1/E1.

8. Méthode de la revendication 1, dans laquelle ledit antigène de HCV est un agrégat E2/E2.

9. Méthode de l'une quelconque des revendications 1 à 8, dans laquelle ledit antigène de HCV ou agrégat est exprimé par un virus de vaccine recombiné.

10. Méthode de l'une quelconque des revendications 1 à 8, dans laquelle ledit antigène de HCV ou agrégat est exprimé dans des cellules CHO.
